# EUROPEAN PATENT APPLICATION

(11) **EP 3 995 171 A2**
(43) Date of publication of application: **11.05.2022**
(21) Application number: 21206379.6
(22) Date of filing: 04.11.2021
(51) Int. Cl.: A61M 37/00

(54) **NEEDLE MODULE WITH MOUTH HAVING FLEXIBLE JAW**

(30) Priority: 06.11.2020 US 202017091928
(71) Applicant: Xiao, Long, Scarborough ON M1P 5G8 (CA)
(72) Inventor: Xiao, Long, Scarborough ON M1P 5G8 (CA)
(74) Representative: Fioravanti, Corrado

(57) **Abstract**

A needle module (100) for a tattoo device includes a housing (40) and a needle bundle (30). The housing comprises a first end for coupling to the tattoo device, a second end comprising a mouth (110), and a channel (420) between the first and second ends. The needle bundle is moveably mounted in the channel of the housing and is reciprocally moveable in an axial direction between a retracted position and an extended position through the mouth. The mouth comprises at least two jaws (10, 20). At least one of the jaws is flexible and flareable when radially biased by the needle bundle.

## Description

### FIELD

The present disclosure relates generally to tattoo devices, particularly to needle modules for tattoo devices.

### BACKGROUND

A tattoo device typically includes a needle for applying ink to skin, a base with a needle actuator, and a needle handle that connects the needle to the base and can be conveniently held in a hand of an operator for manipulating the needle during use. In operation, the tattoo needle is actuated by the needle actuator to reciprocatively move between extended and retracted positions, thereby repeatedly puncturing the subject's skin. Tattoo needles may be provided in a removable needle module, which is attachable to the handle. One or more ink storage cavities may be provided in the needle module for storing ink to be applied to the skin. The needle module is typically sterilized before use and replaced after each use. It is thus convenient to use replaceable and disposable needle modules.

Example tattoo devices and needle modules are disclosed in US 2020/0023175 by Xiao, published January 23, 2020, and US 2019/0217072 by Xiao, published July 18, 2019.

A needle module may have a housing for mounting a bundle of needles therein and a mouthpiece, which has an opening, for allowing the needles to extend outside the housing so they can reach the skin. The bundle of needles may form a generally cylindrical overall profile (referred to as round tattoo needles), or the needles may form one or more rows having a generally rectangular overall profile (referred to as flat tattoo needles). Needles may also form other general profiles, such as weaved magnum needles, stacked magnum needles, round magnums, or curved magnums. The opening in the mouthpiece may have a corresponding shape, such as a circular or diamond shape for round needle bundles or a rectangular shape for row needles. In some cases, the mouthpiece may have a flat open lip and the row needles are exposed on one side and guided on the other side by the open lip. The size of the opening in the mouthpiece is typically selected to accommodate the overall size of the needle bundle or row needles.

Ink may be stored in the needle module, such as between the inner walls of the needle module and the needles. When the needles move back and forth, typically up and down, in the needle module, ink may flow downward to the opening of the mouthpiece and be carried by the needle tip onto the skin. A tattooist may dip the needle module in an ink container to load ink into the needle module.

It is desirable to improve existing tattoo devices and needle module for tattoo devices.

### SUMMARY

Accordingly, an aspect of the present disclosure relates to a tattoo device. The tattoo device comprises a housing comprising a first end for coupling to the tattoo device, a second end comprising a mouth, and a channel between the first and second ends; a needle bundle moveably mounted in the channel of the housing, the needle bundle reciprocally moveable in an axial direction between a retracted position and an extended position through the mouth; wherein the mouth comprises at least two jaws, at least one of the jaws is flexible and flareable when radially biased by the needle bundle.

In selected embodiments, one or more of the following features may be present in the above described needle module, either individually or in any combination. The mouth may comprise two or more circumferentially segmented walls around the needle bundle, and at least one of the walls is flexible and forms the at least one flexible and flareable jaw. At least one of the walls of the mouth may be a guide wall for guiding the needle bundle during reciprocal movement thereof. The guide wall may be rigid, and the at least one flexible wall may be opposite to the guide wall and may bias the needle bundle towards the guide wall. The segmented walls may form a sleeve around the needle bundle and define a tubular channel in the sleeve. The tubular channel may have a generally circular or polygonal radial cross-section. The radial cross-section may be generally rhombus. At least one of the walls may form a generally V-shaped or U-shaped radial profile. The needle bundle may have a generally cylindrical profile and is supported and guided by the at least one wall having the V-shaped or U-shaped radial profile. At least one of the walls may comprise an ink reservoir for storing ink therein. The ink reservoir may comprise one or more grooves, slots, holes, or cavities on an inner surface of the at least one wall. The guide wall may comprise ink storage grooves on an inner surface of the guide wall, and the needle bundle may be biased towards the guide wall to maintain (i) contact with the inner surface of the guide wall and ink stored in the storage grooves, or (ii) a capillary gap between the needle bundle and the inner surface for drawing the ink out of the storage grooves by capillary action. At least two adjacent walls of the segmented walls may be separated by a capillary gap located therebetween, the capillary gap extending parallel to the axial direction and being configured to transfer ink from the ink reservoir to an inner surface of the segmented walls and to optionally store ink therein. The at least one flexible and flareable jaw may comprise a flexible and resilient cantilever attached to the housing. At least one of the jaws may comprise a porous material for storing ink in the porous material. The needle module may comprise a sleeve sleeved over a portion of the jaws and being configured to bias the jaws radially toward the needle bundle. The needle module may comprise a biasing member that biases the needle bundle radially toward the guide wall and axially toward the retracted position. The biasing member may comprise a resilient ring or band. An inner surface of the mouth may be shaped to conform to an external profile of the needle bundle, and the inner surface may be a guide surface for guiding movement of the needle bundle. The needle bundle may comprise a plurality of needles welded to one another. The segmented walls may form an opening at the mouth, at least one of the segmented walls may be distanced from the needle bundle at the opening such that a gap between the at least one segmented wall and the needle bundle is a capillary gap.

Other aspects, features, and embodiments of the present disclosure will become apparent to those of ordinary skill in the art upon review of the following description of specific embodiments in conjunction with the accompanying figures.

### BRIEF DESCRIPTION OF THE DRAWINGS

In the figures, which illustrate, by way of example only, embodiments of the present disclosure:
Fig. 1A is a perspective view of a needle module, illustrative of an embodiment of the present disclosure;
FIG. 1B is an enlarged, partial cross-sectional view of the mouth portion of needle module of FIG. 1A;
FIG. 2 is a side cross-sectional view of the needle module of FIG. 1A, showing the needles in a retracted position;
FIG. 3A is an exploded perspective view of the parts in the needle module of FIG. 1A;
FIG. 3B is a perspective view of an exploded view of partially assembled parts of needle module of FIG. 3A;
FIG. 4 is a perspective view of the mouth in the needle module of FIG. 1A in isolation;
FIG. 5A is a perspective view of the flexible and flareable jaw of the mouth of FIG. 4;
FIG. 5B is a sectional view of the jaw of FIG. 5A;
FIG. 5C is a bottom view of the jaw of FIG. 5A;
FIG. 6A is a perspective view of the rigid jaw of the mouth of FIG. 4;
FIG. 6B is a sectional view of the jaw of FIG. 6A;
FIG. 6C is a bottom view of the jaw of FIG. 6A;
FIG. 7A is a sectional view of the housing in the needle module of FIG. 1A;
FIG. 7B is a perspective view of the housing of FIG. 7B;
FIG. 8 is a partial perspective view of the needle module of FIG. 1A, showing a portion of the mouth and the needles in an extended position;
FIG. 9A is a cross-sectional view of the mouth and the needle bundle in the needle module of FIG. 1A, along line 9a-9a shown in FIGS. 2 and 8;
FIGS. 9B to 9J are cross-sectional views of variations of the mouth and needle bundle in different embodiments;
FIG. 10 is a cross-sectional view of another needle module, illustrative of another embodiment of the disclosure;
FIG. 11A is a perspective view of a needle module with a sleeve over the jaws, illustrative of an embodiment of the disclosure;
FIG. 11B is a perspective view of the sleeve of FIG. 11A in isolation;
FIG. 12 is a perspective cross-sectional view of the needle module of FIG. 11A;
FIG. 13 is a partially cut-off perspective view of an alternative needle module with ink storage grooves;
FIG. 14 is an enlarged view of the cut-off section of FIG. 13;
FIG. 15 is an enlarged top view of the cut-off section of FIG. 14; and
FIG. 16 is a top view of a variation of the cut-off section of FIG. 14.

### DETAILED DESCRIPTION

In brief overview, it has been recognized that it would be beneficial to provide a needle module with a mouth opening that is adjustable to accommodate variations in size and shape of the needle(s) or needle bundle mounted in the needle module. For example, in an embodiment disclosed herein, the mouth of the needle module may have at least one flexible and flareable jaw. The flexible and flareable jaw can conveniently allow the mouth opening to expand or enlarge when radially biased by the needle bundle, such as in situations where the cross-sectional dimension of the needle bundle is larger than the nominal dimension of the mouth opening. The jaws of the mouth can also be configured to continuously apply a radial pressure to the needle bundle.

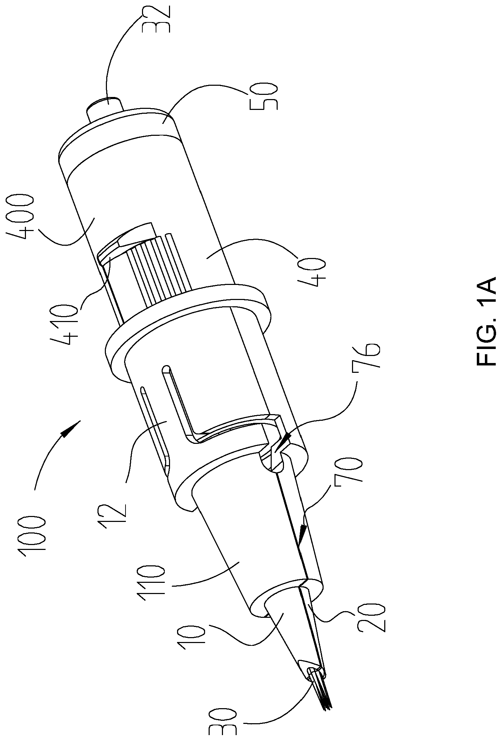

An embodiment of the present disclosure relates to a needle module 100 as illustrated in FIGS. 1A to 8.

As depicted in FIGS. 1A to 3B, needle module 100 includes a needle housing 40 for movably mounting a needle bundle 30 therein. The needle bundle 30 is reciprocally movable along the axial direction in the housing 40. The needle housing 40 has a tubular body portion 400, which defines a tubular longitudinal channel 420 therein and has an upper open end 422 and a lower open end 424. A mouth 110 with an opening 80 is provided at the lower end 424, and a cap 50 is provided at the upper end 422. Mouth 110 is configured to allow the needle tip(s) of needle bundle 30 to extend to outside the needle module 100. The needle housing 40 is configured to engage and couple with a tattoo device (not shown) for applying a driving force to the needle bundle 30, as can be understood by those skilled in the art. For example, as in a typical conventional needle module, the tubular body portion 400 may include a coupling structure 410 for coupling with a tattoo device that has a corresponding coupling structure, such as a handle (not shown) configured to connect a needle module to the tattoo device.

Mouth 110 includes a generally tubular wall which has at least one axially extending cut 70 so the wall is segmented into two wall segments, wall 11 and wall 21, and there is an axially extending gap between the two adjacent wall segments. In different embodiments, the segmented wall may include two or more segmented wall sections, as will be further described below.

At least one of the segmented wall sections, such as wall 21, may provide a guide surface for guiding movement of the needle bundle 30 and optionally for feeding ink to the needle bundle 30, as will be described further below.

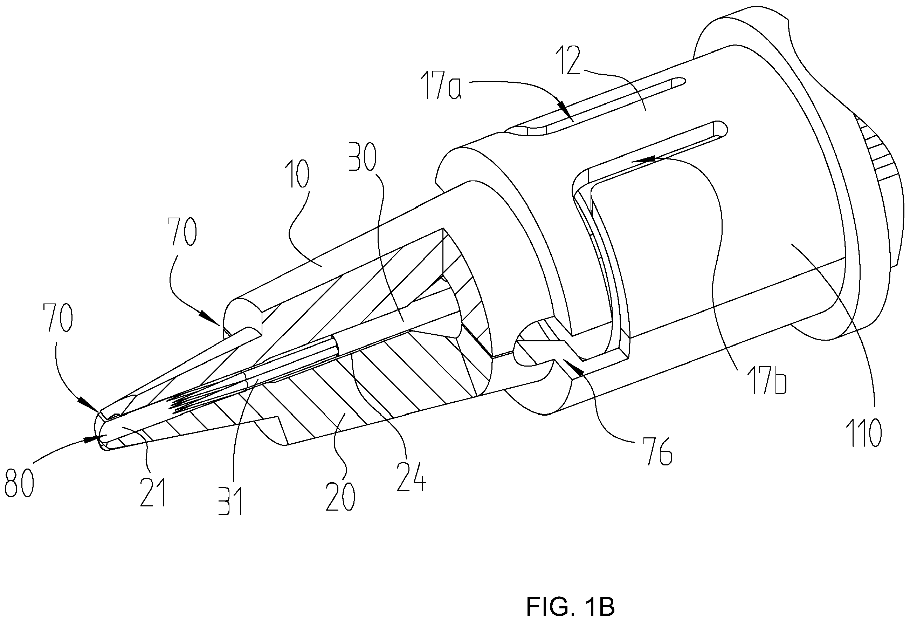

The needle bundle 30 can move through the mouth 110 between a retracted position (see FIG. 1B) and an extended position (see FIG. 1A).

Each of mouth 110 and cap 50 may be independently configured in a suitable manner for connecting with and engaging the lower end or the upper end of body portion 400 respectively, with any suitable engagement or locking mechanism. For example, they may be engaged by tabs, threads, clamps, pins, keys, and corresponding openings, notches, threads, holes, keyways, or the like as can be understood by those skilled in the art. As depicted, both mouth 110 and cap 50 may frictionally engage the inner walls of body portion 400, and are interlocked in the mounted positions with a tab.

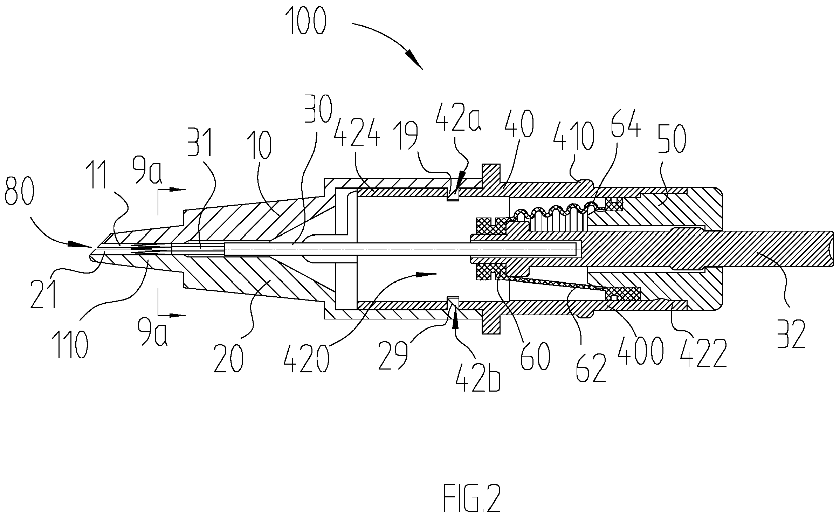

For example, as illustrated in FIG. 2, the housing body portion 400 may include mating notches 42a, 42b (also individually or collectively referred to as 42) at the lower end 424, and the mouth 110 may include corresponding mating stabs 19,29 that matches and mates with the mating notches 42 for orienting/aligning and positioning the mouth 110 in relation to the housing body portion 400.

As will be appreciated and as in conventional needle modules, needle bundle 30 is housed and guided in housing 40 in a manner to allow the needle bundle 30 to reciprocally move up and down during use. The detailed construction and mechanism for such mounting and reciprocal movement are not the focus of this disclosure, and can be implemented by a skilled person in the art according to known techniques or constructions, except in aspects specifically described below. Thus, these details will not be discussed herein.

It is noted that, however, to facilitate the reciprocal movement of the needle bundle 30, a biasing member 60 such as a resilient band may be provided and configured to pull the needle bundle 30 up during each movement cycle after the needle bundle 30 is pushed down by an actuating or driving mechanism (not shown). In an embodiment in the present enclosure, the biasing member 60 is not only configured and used to facilitate reciprocal movement of the needle bundle 30, but also configured and used to bias the needle bundle 30 toward a guide surface and an ink reservoir in the mouth 110 to increase or maintain contact between the needle bundle 30 and ink stored in the ink reservoir. Further details of the needle driving mechanism and the biasing member for driving and biasing the needle bundle 30 may be found in US 2020/0023175, US 2019/0217072, and CA 3011593, and the references cited therein, the relevant contents in each of which are incorporated herein by reference.

As better illustrated in FIG. 2, cap 50 has an opening to allow a drive shaft, such as shaft 32 or a further shaft (not shown), to pass through and reciprocally move therein. The drive shaft 32 transmits the needle actuating force to the needle bundle 30. Typically, a needle actuator (not shown) is used to apply a downward (right-to-left in FIG. 2) force via drive shaft 32 to push the needle bundle 30 downward towards the extended position, but the needle actuator will not apply an upward force to pull the needle bundle 30 back to the retracted position. Instead, the biasing member 60 is provided in the needle module 100 to pull the needle bundle 30 back to the retracted position, such as through shaft 32. Biasing member 60 may have a first side 62 and a second side 64 with different resiliency forces for producing a radial biasing component, so that biasing member 60 can both bias the needle bundle upward and radially toward the guide surface on jaw 20.

The needle bundle 30 includes needles 31. When the tips of the needles 31 in the needle bundle 30 extend to the extended position through the mouth 110 outside the housing 40, the tips of the needles 31 can penetrate the skin and apply the ink to the skin to form tattoo patterns.

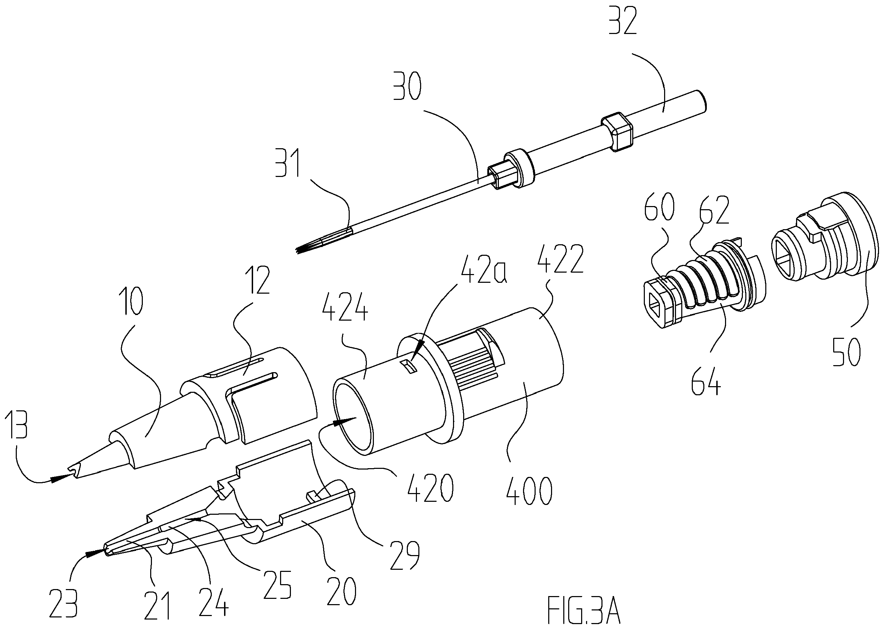

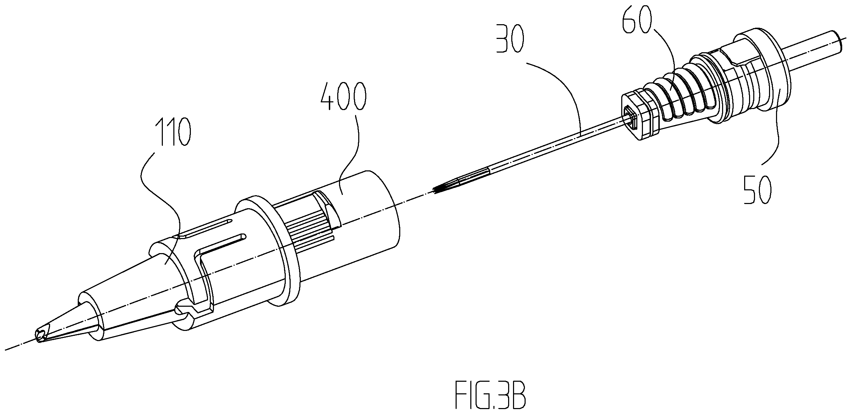

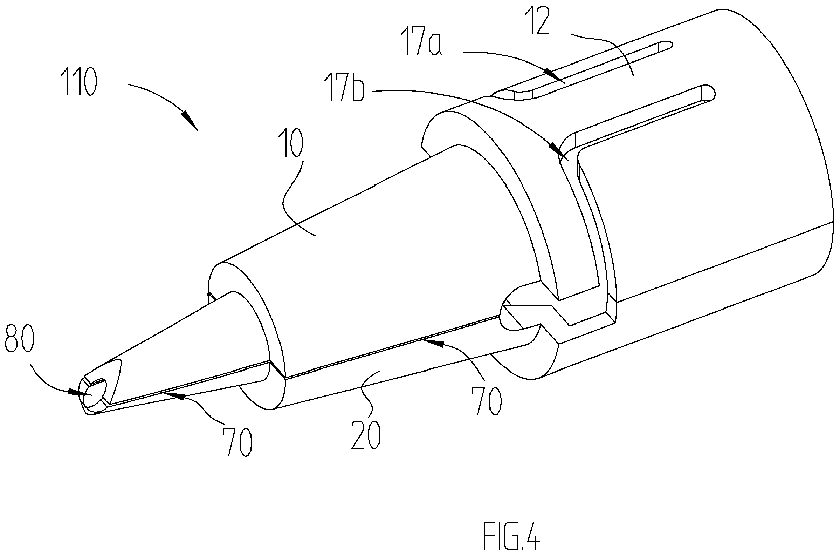

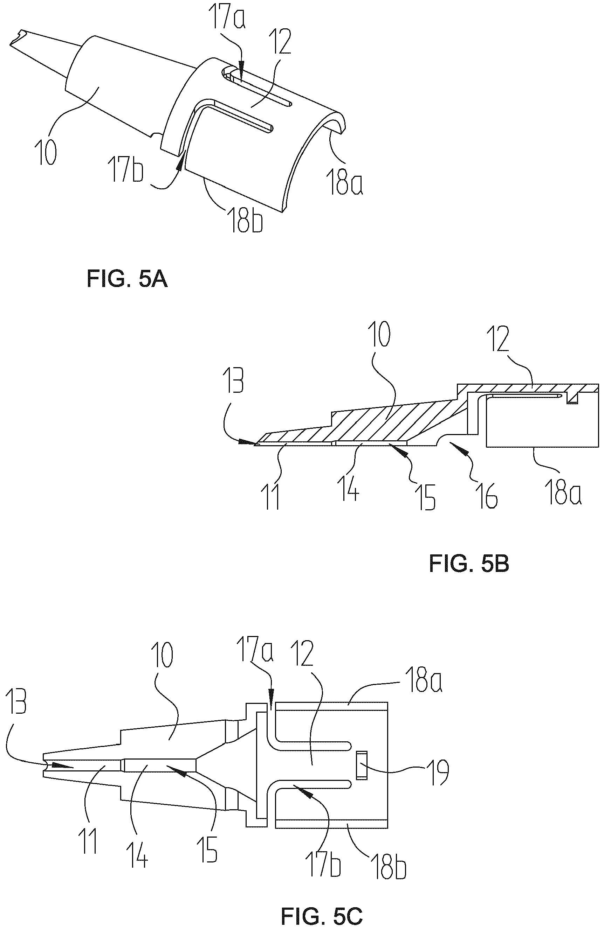

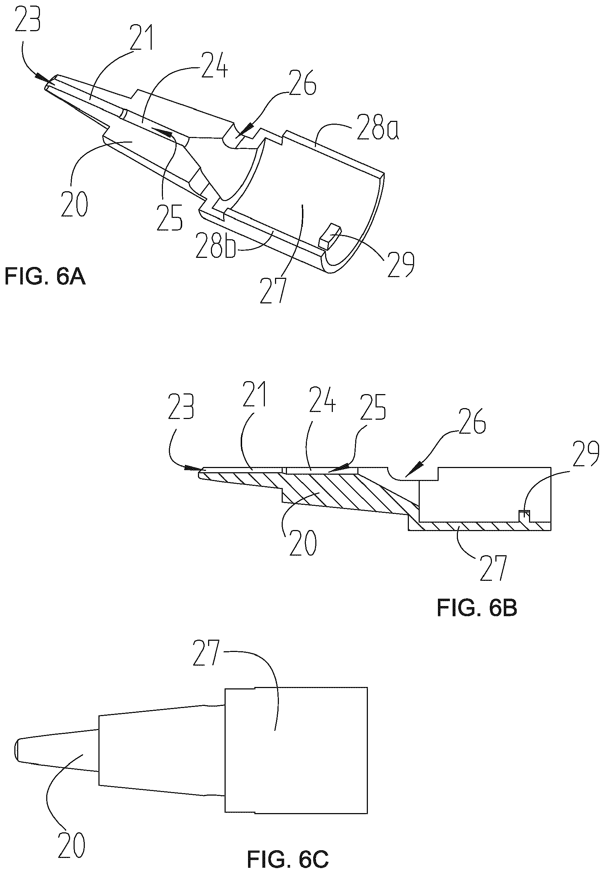

As better illustrated in FIGS. 3A, 3B, and 4, mouth 110 includes two segmented wall portions 10 and 20, which may be considered as jaws of the mouth 110 (and are thus referred to as jaw 10 and jaw 20 hereinafter). Jaw 10 is further illustrated in FIGS. 5A, 5B and 5C. Jaw 20 is further illustrated in FIGS. 6A, 6B and 6C.

Each jaw 10 or 20 has corresponding mating surfaces 18a, 18b and 28a, 28b respectively, for mating with the other jaw 10 or 20. When assembled, mating surfaces 18a, 18b and 28a, 28b may engage each other to connect the two Jaws 10, 20. Jaws 10, 20 may be made of a plastic material such as a polycarbonate (PC). Jaws 10, 20 may be connected or mated at surfaces 18a, 18b and 28a, 28b by an adhesive, or welding, such as ultrasound welding. Jaws 10, 20 may be similarly connected to end 424 of the housing body 400 by an adhesive, or welding, such as ultrasound welding. Jaw 10 also defines a through groove with a narrower portion 13 and a wider portion 15. Similarly, jaw 20 defines a through groove with a narrower portion 23 and a wider portion 25.

Referring to FIGS. 2, 5A-5C, and 6A-6C, when jaws 10, 20 are assembled and mounted on the tubular body portion 400, the aligning stab 19 on jaw 10 is inserted into notch 42a of the tubular body portion 400, aligning stab 29 of jaw 20 is inserted into notch 42b of the tubular body portion 400, so the mouth 110 is aligned and secured in position.

Jaw 10 includes the wall segment 11. As depicted, segment 11 is arched and form a half circle. Jaw 20 similarly includes an arched wall segment 21. When mated, walls 11 and 21 of Jaws 10 and 20 form a generally cylindrical channel having a circular cross-section profile.

When jaws 10 and 20 are braced toward each other, grooves 15 and 25 form a channel that allows the needle(s) on the needle bundle 30 to pass through. The sizes of the grooves 15 and 25 are selected so that the channel formed by them has a larger size than the size of the opening 80. Grooves 13 and 23 also form a channel and defines the mouth opening 80 that allows the needle tip(s) of the needle(s) 31 on the needle bundle 30 to pass through. As grooves 13 and 23 are narrower than grooves 15, 25, the channel formed by grooves 13 and 23 has a smaller size than the size of the channel formed by grooves 15 and 25.

While jaws 10, 20 are connected at surfaces 18a, 18b and 28a, 28b, walls 11 and 21 are separated by an axially extending gap 70. As used herein, a gap is considered a "cut", and a "cut" may be formed by cutting the material or may form by bringing two segments close to each other. The cut is not necessarily formed by cutting. The cut may be a slit or narrow gap. In some embodiments, the cut forms a capillary gap.

In some embodiments, needle bundle 30 may include a plurality of needles 31. In some embodiments, needle bundle 30 may include a single needle 31. For example, in a particular embodiment, needle bundle 30 may include seven (7) needles. The needles may form a generally round cross-sectional profile (round needles). The needles in the needle bundle 30 may be welded together such as by lead-free soldering. The needles in needle bundle may be attached to needle drive shaft 32 such as by bonding with an adhesive or by a suitable coupling mechanism.

In some embodiments, the specified diameter of the needle bundle 30 may be closely matching the specified diameter of the opening 80 in mouth 110, which is defined by Jaws 10 and 20. The specified diameter of opening 80 may be slightly larger than the specified diameter of needle bundle 30. However, in practice, the actual sizes of needle bundle 30 may vary and its radial cross-section at different axial locations may not be perfectly circular due to various reasons, so the shapes of the needle bundle 30 and the opening 80 may not exactly match and the needle bundle 30 may have a section that presses against the opening creating increased friction. The variation in the needle bundle dimensions may result from design tolerance, manufacturing tolerance, assembling tolerance, or the like. For example, each needle may have a varying diameter or size, the packing of the needles may vary, the relative arrangement between the needles may vary, and the soldering material left in the needle bundle between the needles may also vary. It is therefore quite often in practice the diameter of the needle bundle is not exactly as specified by the manufacturer.

On the one hand, when the actual size of the needle bundle 30 is smaller than its specified size and the size of the opening 80, the needle bundle can still pass through the mouth 110, but may tend to wabble during movement as the space between the opening 80 and the needle bundle is larger and allows more radial movement. On the other hand, when the size of the needle bundle 30 is larger than the specified size or the size of the opening 80, if the opening 80 is rigid, the needle bundle 30 may not be able to pass through opening 80, or a large friction may result due to the radial force existing between the needle bundle 30 and the wall forming opening 80. The friction may impede movement of the needle bundle 30 through the opening 80, generate excessive heat, or result in increased wear and tear.

In the present embodiment, however, one or both jaws 10 and 20 are flexible and can flare, or bend outward, under radial pressure from the needle bundle 30. As a result, the opening 80 is enlarged and the friction between the walls and the needle bundle can be substantially reduced and the needle bundle can smoothly move through the opening 80 back and forth. The flexible jaw(s) may be made of a flexible material such as a bendable or resilient plastic or metallic material, or may be made of a rigid material but have a structure or mechanism that allows the jaw to flare under radial pressure, as illustrated herein.

Thus, the flareable jaw(s) allow(s) the mouth 110 to accommodate different needle bundle with variable sizes within an increased range, and still provide suitable support and guidance for the needle bundle. In particular, the needle bundle 30 may still maintain contact with the guide surface on the wall and the ink provided on or in the guide surface, as will be further described below.

In some embodiments, to allow the flexible jaw 10 to flare more, a less flexible wall section is attached to a more flexible cantilever 12 as illustrated in FIGS. 5A-5C.

The cantilever 12 may be formed by providing two through cuts 17a, 17b, in the wall of jaw 10 as illustrated in FIG. 5A. the cantilever 12 between the two cuts 17a, 17b has a smaller width and thickness than the attached wall section and is thus easier to bend and flare. A base end of the cantilever 12 of jaw 10 is fixedly attached to the housing body 400 at the stab 19. The wider wall section of jaw 10 is attached to a distal end of the cantilever 12 and is thus free hanging. The terminal and wider section of jaw 10 is held in place only by cantilever 12. When cantilever 12 flexes radially under pressure, the terminal and wider section of jaw 10 flares radially to a greater extent. Thus, with the cantilever structure, the jaw 10 can conveniently flare to a relatively large extent under a relatively small biasing force.

The distance between the cuts 17a, 17b may be adjusted to adjust the resistance to flexing and the flexing extent of the cantilever 12. The cantilever 12 between the cuts 17a, 17b may be generally flat and relatively thin. The width and thickness of the cantilever 12 may be selected to provide the required flexibility while maintain the desired or needed mechanical strength and integrity.

In the embodiment depicted in FIG. 6A-6C, the jaw 20 does not have a cantilever structure. Rather, jaw 20 is rigid and stable, and provides a guiding surface for supporting and guiding the movement of the needle bundle 30. The backing section 27 of Jaw 20 does not have any cuts thereon and thus provides strong and stable support for wall 21 so the position of wall 21 is relatively stable and unmovable with respect to the housing body 400.

As illustrated in FIGS. 1A and 1B, between the ends of cuts 70, 17a, 17b, an air opening 76 may be provided. As illustrated in FIGS. 5A, 6A and 6B, each air opening 76 may be formed by opening 16 on jaw 10 and opening 26 on jaw 20.

The biasing member 60 biases the needle bundle 30 toward jaw 20 and the guide surface thereon.

When the needle bundle 30 has a relatively lager size, jaw 10 also biases the needle bundle 30 towards jaw 20 and the guide surface thereon.

In an alternative embodiment, jaw 20 may also have a cantilever structure, similar to Jaw 10, in which case both jaws 10 and 20 may flare to enlarge the mouth opening 80.

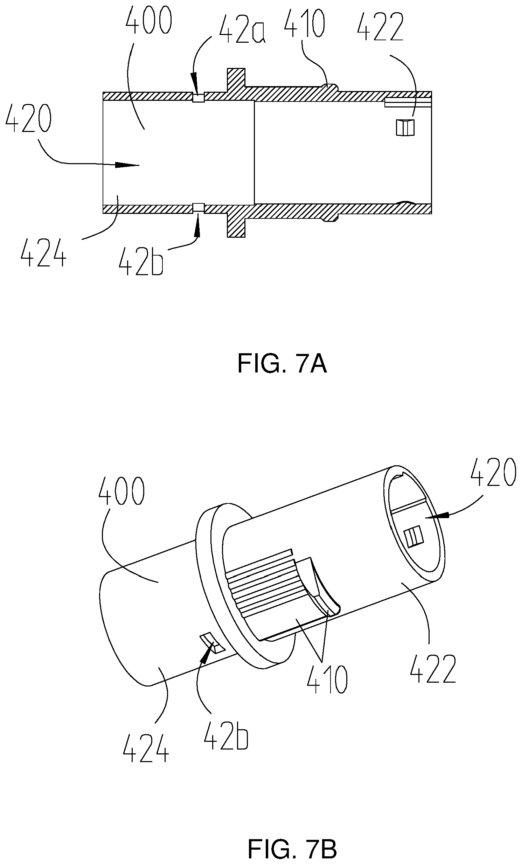

FIGS. 7A and 7B show the housing body 400 in isolation. As can be seen, notches 42a and 42b may be generally rectangular in cross-section.

The coupling structure 410 may include any suitable coupling or mating structure, such as tabs, ribs, or keyways, as can be understood by those skilled in the art.

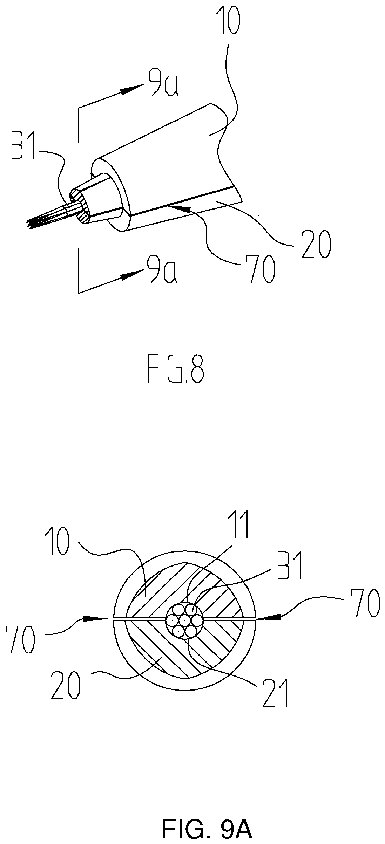

FIG. 8 shows a portion of the mouth 110 with tips of the needles 31 in the extended position.

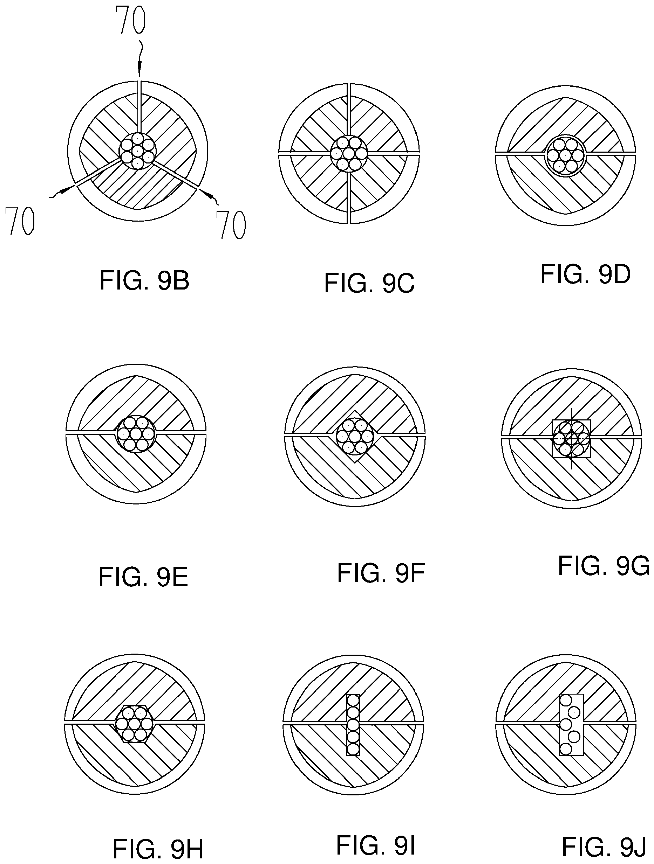

FIG. 9A is an axial cross-sectional view of the mouth 110 and needles 31 of the needle module 30. As can be seen, wall 11 of Jaw 10 and wall 21 of Jaw 20 are segmented by cuts 70. Because the diameter of the circular opening between walls 11 and 21 are substantially the same as the overall diameter of the needle bundle 30, the walls 11 and 21 are in contact with needles 31 of the needle bundle 30.

As can be seen in FIGS. 5B, 6B and 9A, walls 11 and 21 form jaws of the mouth 110, which engages and holds needle bundle 30 to limit its radial movement. The walls 11 and 21 also can be considered to form a sleeve around the needle bundle and define a tubular channel in the sleeve. The tubular channel may be generally cylindrical or prismatic. The tubular channel may have a generally circular or polygonal radial cross-section. The radial cross-section may be generally rhombus. At least one of the walls may form a generally V-shaped or U-shaped radial profile.

The needle bundle 30 has a generally cylindrical profile and is supported and guided by both of walls 11 and 21 (also referred to as jaws 11 and 21 herein).

FIGS. 9B to 9J show different embodiments of the jaws of mouth 110 and possible profiles of the needle bundle 30 and the cross-sectional profiles of opening formed by the jaws. For example, mouth 110 may have three jaws separated by three cuts 70 as illustrated in FIG. 9B, or four jaws separated by four cuts as shown in FIG. 9C. The needle bundle 30 may have a smaller diameter and be distanced from the opening formed by the jaws by a gap as shown in FIG. 9D. The opening in mouth 110 formed by the jaws may also have a generally oval or elliptic profile as shown in FIG. 9E, or have a polygonal profile such as a rhombus (or diamond), square, hexagon, or rectangular profile as illustrated in FIGS. 9F to 9J respectively. As shown in FIG. 9F, each of jaws 11 and 21 may have a generally V-shaped inner surface. The V-shaped inner surface may be conveniently used to support and guide the needle bundle 30. With a generally V-shaped guide surface, the generally cylindrical needle bundle 30 may be more stably supported and guided, as the V-shaped surface biases the needle bundle 30 form both sides and can prevent lateral movement or vibration of the needle bundle 30. As illustrated, the opening in the mouth 110 may be circular or have a number of straight sides, such as 4 or 6 sides. As illustrated in FIGS. 9I and 9J, the needles in needle bundle 30 may have different arrangements or patterns, and may form an array or a matrix, or may be aligned linearly or offset laterally. As illustrated, the mouth 110 may include two or more jaws, at least one of which is flexible or can flare under radial pressure. All of the jaws of the mouth 110 may be flexible in some embodiments. In other embodiments, one or more of the jaws may be rigid or non-flexible.

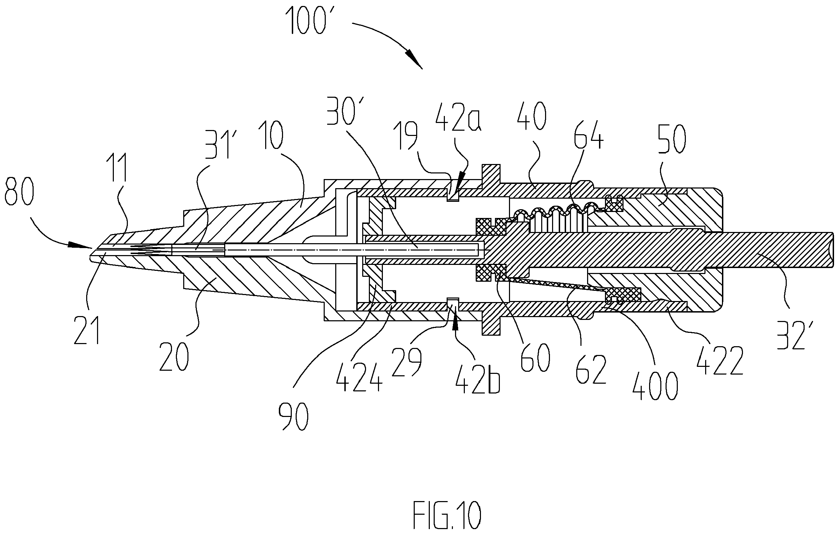

Another embedment is illustrated in Fig. 10, which shows a needle module 100'. Needle module 100' is similar to needle module 100 but has an additional component, slidable bearing support 90. Bearing support 90 has a bearing element disposed inside the housing body 400 and supports a needle bundle 30' through a drive shaft 32' in the housing body 400. Bearing support 90 has a central opening and allows drive shaft 32' with needle bundle 30' to slide back and forth in the central opening. As depicted, needle bundle 30' is attached to, and supported by, drive shaft 32', and drive shaft 32' is in turn supported at two locations, the bearing support 90 and cap 50. Thus, needle bundle 30' does not need to be supported at the mouth 110 or the mouth opening in the mouth 110. When the needle bundle 30' is not supported at the mouth, the jaws, or segmented walls, of the mouth 110 may be all flexible, such as being formed from a resilient or elastic material. In this case, it is not necessary any of the jaws or walls in the mouth 110 is rigid. In such cases, Jaw 20 of the mouth 110 may also have cuts or channels, similar to cuts 17a, 17b in Jaw 10 as discussed above.

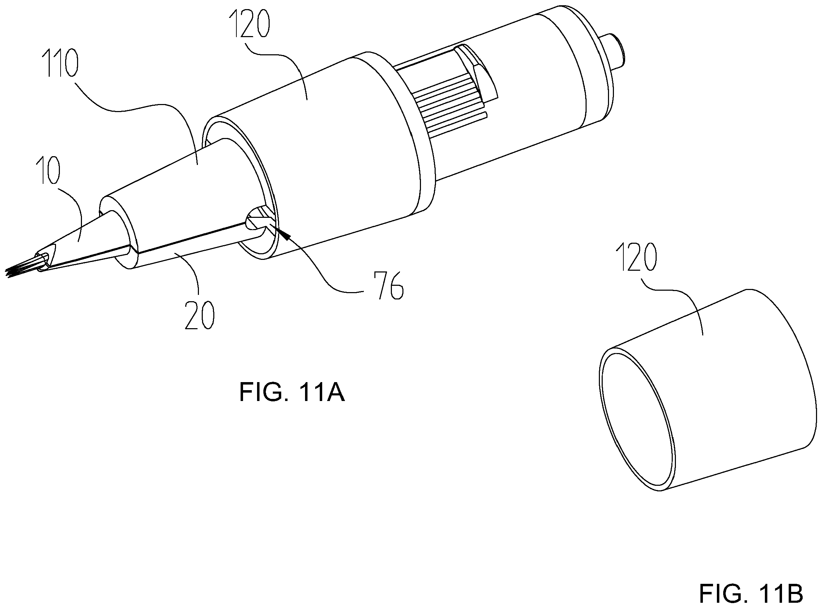

FIG. 11A shows a needle module having a constriction sleeve 120.

FIG. 11B shows the constriction sleeve 120 in isolation, which may be formed of an elastic material, such as rubber or silicone.

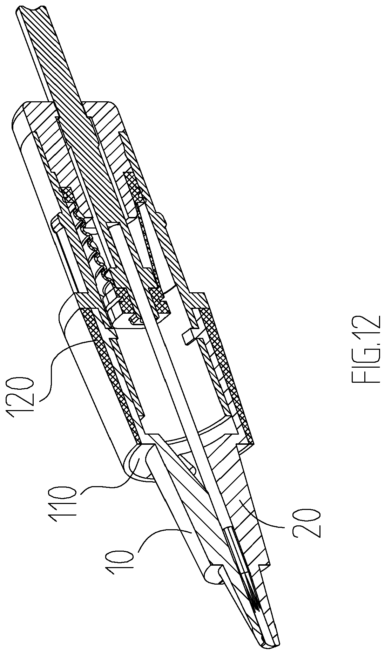

Fig.12 shows a cross-section view of the needle module of FIG. 11A. The constriction sleeve 120 surrounds Jaws 10 and 20 and biases them towards each other. Constriction sleeve 120 is sleeved over cuts 17a and 17b, and indirectly biases needles 31, through wall 11, towards wall 21.

When the needle bundle 30 moves at a high speed, the air in the internal compartments of the needle module can be locally pressurized and compressed due to the movement of the needle bundle 30 and the biasing member 60. The pressurized or compressed air in the needle module, particularly when the pressure is quickly increased, can drive ink stored in the needle module 100 out of the needle module 100 through openings such as mouth opening 80 and other openings of the mouth 110. The pressures in the needle module may also fluctuate due to the needle bundle acting as a piston and the and the biasing member 60 acting as bellows, both pressurizing the air in the housing body. These effects can increase the risk of accidentally or unintentionally discharging ink, and thus impeding normal operation of the tattoo device. To address this potential problem, air openings 76 may be provided to reduce the internal air pressure build-up in the needle module housing. Two air openings 76 may be provided, each at an end of the respective cuts 70, or the intersections of cuts 70 and 17a, 17b. For example, each side of the mouth 110 may be provided with an air opening 76 as described earlier. The air openings, also referred to as breathing holes, allow the internal pressure in the needle module to be maintained at, or quickly returned to, the level of the external air pressure. Constriction sleeve 120 embraces Jaws 10 and 20, biases Jaws 10 and 20 toward each other, and presses the base portions of jaws 10 and 20 against the housing body 400.

In this embodiment, connection surfaces 18a, 18b and connection surfaces 28a, 28b do not need to be bonded or welded together, and they can be braced toward each other or together by constriction sleeve 120.

In some embodiments, with the constriction sleeve 120, Jaws 10 and 20 do not need to have cuts 17a and 17b, as the constriction sleeve 120 can be resilient and deformable and thus apply a variable pressure onto the jaws to allow one or both of the jaws 10, 20 to flare or retract.

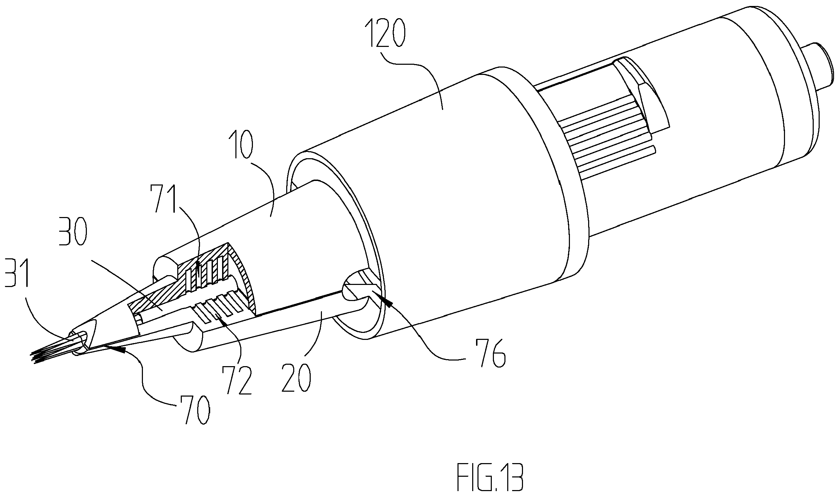

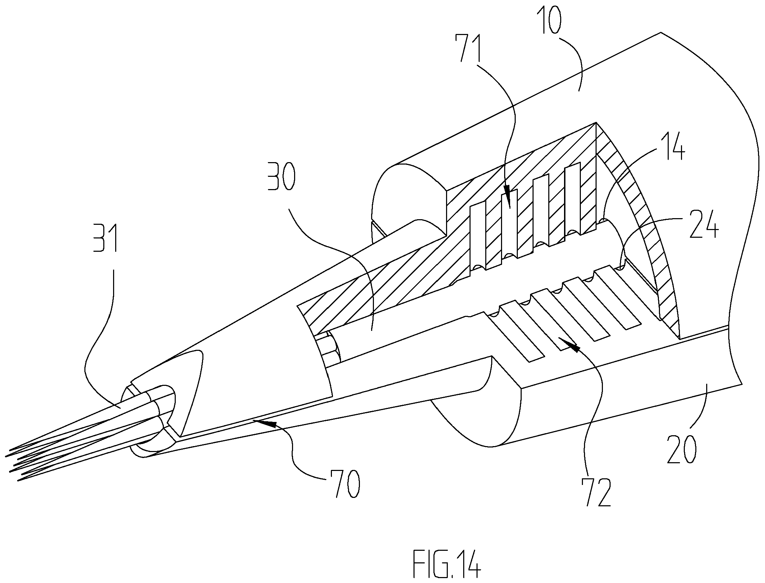

One or more of jaws 10, 20 may include an ink storage or ink reservoir. For example, as illustrated in FIGS. 13, 14 and 15, in an alternative embodiment, each of jaws 10, 20 may have ink storage grooves 71, 72 for storing ink therein. Grooves 71, 72 may be sized so that ink can be held in the grooves by capillary action. As depicted, grooves 71, 72 extend laterally and generally perpendicular to the axial direction or the direction of the needle bundle movement. The needle(s) 31 on needle bundle 30 may contact the inner surfaces of jaws 10, 20, or close to, and come into contact with the ink stored in the needle module, and draw the ink out of the storage grooves 71, 72 by capillary action.

The inner surface of jaw 20, and possibly the inner surface of jaw 10, can provide a guide surface to support and guide the needle bundle 30 along the channels formed by grooves 13, 23. The needle bundle 30 may be biased towards the inner surface 24 at groove 25, as described elsewhere herein.

Alternatively, the needle bundle 30 may be supported and guided at the mouth opening 80, and does not contact the inner surfaces of groove 15 and 25.

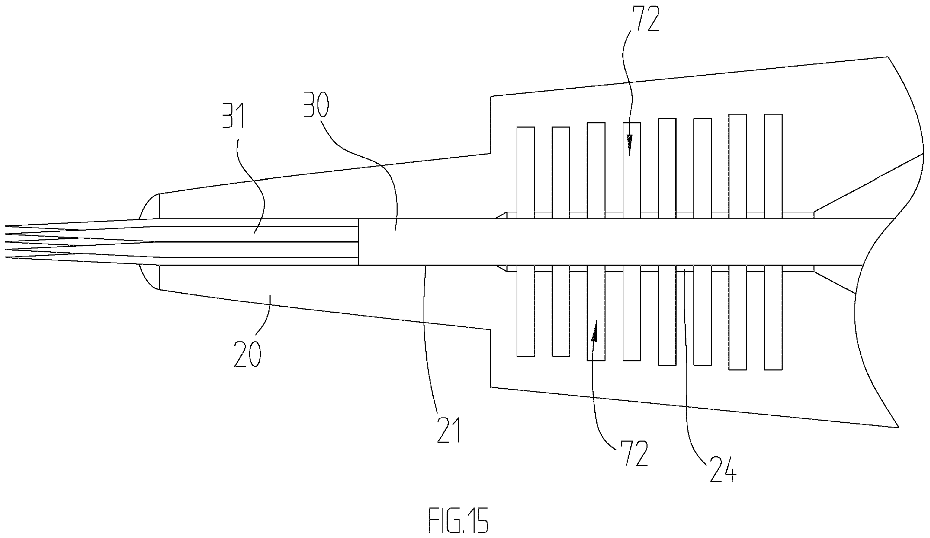

In some embodiments, as illustrated in FIG. 15, the inner surface 24 in groove 25 may be recessed and do not contact the needles 31. In this case, the inner surface 24 at groove 25 does not support and does not guide the movement of the needles 31. Although a gap exists between the inner surface 24 and the needles 31, the gap may be filled with ink, and provide an ink reservoir. The gap may be about 0.1 mm to about 0.3 mm, and may be capillary gap. Due to the capillary action, the ink may be better stored and will not easily or uncontrollably flow downward due to gravity.

As illustrated in FIGS. 13, 14 and 15, ink storage grooves 71, 72 are formed on walls 14, 24 respectively. Grooves 71, 72 may be about 0.2 mm to about 0.5 mm wide. It is noted that cut 70 may have a smaller width but may also be a capillary gap. Cut 70 may connect grooves 71, 72 and allow fluid communication between different grooves 71, 72 through cut 70. Cut 70 may also conveniently function as a conduit for transferring the ink towards the opening 80 and wetting the needle tips at the opening 80.

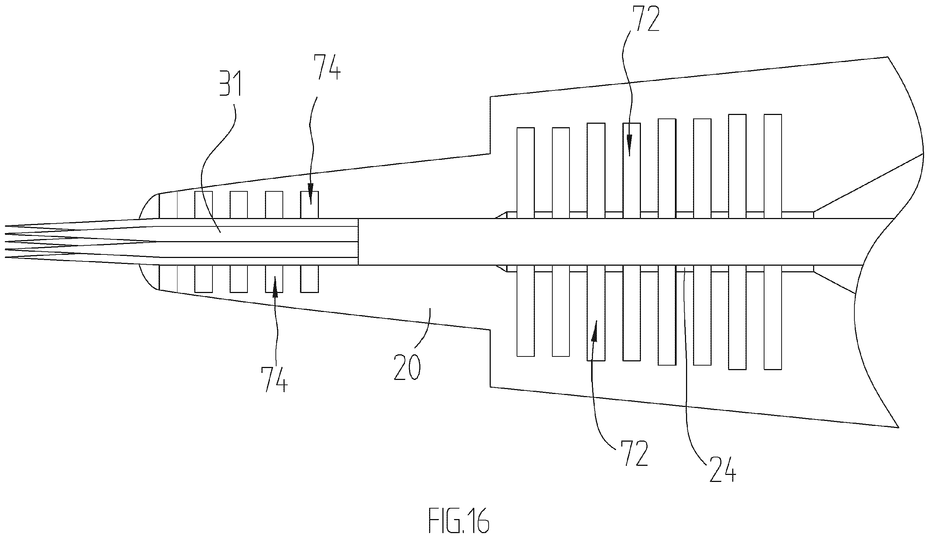

FIG. 16 illustrates a different embodiment, where transversal ink storage grooves 72 are provided at longitudinal grooves 15, 25 and 74 are provided at longitudinal grooves 13, 23. Grooves 72, 74 may also form capillary gaps for storing ink by capillary forces. The cut 70 (not shown in FIG. 16) may connect grooves 72 with grooves 74 to provide fluid communication therebetween. In this case, the needle tips of needles 31 may be more evenly wetted with the ink at grooves 74.

Ink storage grooves 74 can be used to control the dispensing of ink in the needle module, reduce the risk of spilling ink or splashing of ink. Ink stored in grooves 71, 72 may be transferred to grooves 74 through cut 70 continuously.

Ink storage grooves 71, 72, 74 may be structured and configured as disclosed in US 2013/0226211 and US 2017/0072178, the relevant contents of each of which are incorporated herein by reference.

When the cross-sectional size of the needle bundle 30 is smaller than the size of the mouth opening 80, the gap between the needle bundle 30 and the mouth opening 80 may be filled with ink flown from grooves 71, 72. Similarly, when the mouth opening has a profile or shape that is different from the shape of the needle bundle 30, such as when the mouth opening has a square, rectangular, or diamond profile or shape, a gap also exists between the needle bundle 30 and the wall surface at or near the opening 80, which may be similarly used to transfer ink from grooves 71, 72 to the opening 80.

In some embodiments, one or more of the jaws of the mouth 110 may be made of a porous material for storing ink therein. The pores in the porous material may be filled with ink, like grooves 71, 72, 74. One or more of the jaws of the mouth 110 may also have multiple grooves for storing ink. In some embodiments, two or more cuts 70 may also be filled with ink to store the ink, in addition to being used as a communication channel for transporting ink.

As can be appreciated, with a flexible and flareable jaw in the mouth of the needle module, the mouth opening can adjust to more closely match the actual dimension and shape of the needle bundle, which may lead to one or more beneficial effects. For example, a wider variation in the needle bundle dimensions and shapes may be tolerated. Friction and vibration, or jamming of the needles, in the needle module during use may be reduced. Loss of ink due to excessive ink gushing or splashing out of the mouth opening can be reduced, limited or prevented, which may in turn reduce or prevent ink tainting on the subject skin or injuring the eyes or the surrounding skin of the subject being tattooed.

It should be understood that as used herein, the term "tattoo" can refer to cosmetic tattoo or permanent makeup, and a tattoo device or machine may refer to devices or machines for applying cosmetic tattoo or permanent makeup.

It should also be understood that as used herein, the phrase "flexible jaw" refers to a jaw that is moveable or bendable so it can flare under outward radial pressure. The flexible jaw may be moveable due to its structure or material, or both. For example, the flexible jaw may be made of a semi-rigid material, or a material that is normally considered rigid, but has a thin or narrowed section or end such that the terminal end of the jaw can flare under a lateral pressure. The flexible jaw may include a resilient material or be biased to apply a radially inward pressure on the needle bundle in contact with the jaw, and to resume its original or normal position or shape after the needle bundle retracts or when a needle bundle with a smaller cross-section size is used. As can be appreciated, the flexible jaw is flexible to a desired extent to allow thicker needle bundles to pass through the mouth, but it should have sufficient mechanical strength and rigidity to maintain the mouth structure.

It should also be understood that modifications and variations to the specific embodiments described above are possible.

### CONCLUDING REMARKS

It will be understood that any range of values herein is intended to specifically include any intermediate value or sub-range within the given range, and all such intermediate values and sub-ranges are individually and specifically disclosed.

It will also be understood that the word "a" or "an" is intended to mean "one or more" or "at least one", and any singular form is intended to include plurals herein.

It will be further understood that the term "comprise", including any variation thereof, is intended to be open-ended and means "include, but not limited to," unless otherwise specifically indicated to the contrary.

When a list of items is given herein with an "or" before the last item, any one of the listed items or any suitable combination of two or more of the listed items may be selected and used.

Of course, the above described embodiments of the present disclosure are intended to be illustrative only and in no way limiting. The described embodiments are susceptible to many modifications of form, arrangement of parts, details and order of operation. The invention, rather, is intended to encompass all such modification within its scope, as defined by the claims.

## Claims

1. A needle module for a tattoo device, comprising:
a housing comprising a first end for coupling to the tattoo device, a second end comprising a mouth, and a channel between the first and second ends;
a needle bundle moveably mounted in the channel of the housing, the needle bundle reciprocally moveable in an axial direction between a retracted position and an extended position through the mouth;
wherein the mouth comprises at least two jaws, at least one of the jaws is flexible and flareable when radially biased by the needle bundle.

2. The needle module of claim 1, wherein the mouth comprises two or more circumferentially segmented walls around the needle bundle, and at least one of the walls is flexible and forms the at least one flexible and flareable jaw.

3. The needle module of claim 2, wherein at least one of the walls is a guide wall for guiding the needle bundle during reciprocal movement thereof.

4. The needle module of claim 3, wherein the guide wall is rigid, and the at least one flexible wall is opposite to the guide wall and biases the needle bundle towards the guide wall.

5. The needle module of any one of claims 2 to 4, wherein the segmented walls form a sleeve around the needle bundle and define a tubular channel in the sleeve, wherein the tubular channel optionally has a generally circular or polygonal radial cross-section.

6. The needle module of any one of claims 2 to 5, wherein at least one of the walls form a generally V-shaped or U-shaped radial profile, wherein, optionally, the needle bundle has a generally cylindrical profile and is supported and guided by the at least one wall having the V-shaped or U-shaped radial profile.

7. The needle module of any one of claims 2 to 6, wherein at least one of the walls comprises an ink reservoir for storing ink therein, the ink reservoir comprising one or more grooves, slots, holes, or cavities on an inner surface of the at least one wall.

8. The needle module of claim 7, wherein at least two adjacent walls of the segmented walls are separated by a capillary gap located therebetween, the capillary gap extending parallel to the axial direction and being configured to transfer ink from the ink reservoir to an inner surface of the segmented walls and to optionally store ink therein.

9. The needle module of claim 3 or claim 4, wherein the guide wall comprises ink storage grooves on an inner surface of the guide wall, and the needle bundle is biased towards the guide wall to maintain (i) contact with the inner surface of the guide wall and ink stored in the storage grooves, or (ii) a capillary gap between the needle bundle and the inner surface for drawing the ink out of the storage grooves by capillary action.

10. The needle module of any one of claims 1 to 9, wherein the at least one flexible and flareable jaw comprises a flexible and resilient cantilever attached to the housing.

11. The needle module of any one of claims 1 to 10, wherein at least one of the jaws comprises a porous material for storing ink in the porous material.

12. The needle module of any one of claims 1 to 11, comprising a sleeve sleeved over a portion of the jaws and being configured to bias the jaws radially toward the needle bundle.

13. The needle module of claim 3 or claim 4, comprising a biasing member that biases the needle bundle radially toward the guide wall and axially toward the retracted position, wherein the biasing member preferably comprises a resilient material.

14. The needle module of any one of claims 1 to 13, wherein an inner surface of the mouth is shaped to conform to an external profile of the needle bundle, and the inner surface is a guide surface for guiding movement of the needle bundle.

15. The needle module of any one of claims 2 to 9, wherein the segmented walls form an opening at the mouth, at least one of the segmented walls is distanced from the needle bundle at the opening such that a gap between the at least one segmented wall and the needle bundle is a capillary gap.
